# EUROPEAN PATENT SPECIFICATION

(11) **EP 2 785 336 B1**
(45) Date of publication and mention of the grant of the patent: **30.09.2015**
(21) Application number: 12795340.4
(22) Date of filing: 29.11.2012
(51) Int. Cl.: A61K 31/08, A61P 17/00, A61P 17/12

(54) **USE OF PEGYLATED ALCOHOLS FOR THE TREATMENT OF ACTINIC KERATOSIS**
VERWENDUNG VON PEGYLIERTEN ALKOHOLEN ZUR BEHANDLUNG VON AKTINISCHER KERATOSE
UTILISATION D'ALCOOLS PÉGYLÉS POUR LE TRAITEMENT DE LA KÉRATOSE ACTINIQUE

(30) Priority: 30.11.2011 EP 11191387; 30.11.2011 US 201161564893 P
(43) Date of publication of application: 08.10.2014
(73) Proprietor: Merz Pharma GmbH & Co. KGaA, 60318 Frankfurt am Main (DE)
(72) Inventor: BARG, Heiko, 69469 Weinheim (DE); POOTH, Rainer, 65812 Bad Soden/Taunus (DE); ABTS, Harry Frank, 61440 Oberursel (DE); KIEHM, Kevin, 48291 Telgte (DE)
(74) Representative: Michalski Hüttermann & Partner Patentanwälte mbB
(86) International application number: PCT/EP2012/004943
(87) International publication number: WO 2013/079211

(56) References cited:
- WO-A1-2010/080345
- WILLIAM J MCINTYRE ET AL: "Treatment Options for Actinic Keratoses", American Family Physician , vol. 76, no. 5 2007, pages 667-671, XP002667626, Retrieved from the Internet: URL:http://www.aafp.org/afp/2007/0901/p667 .html [retrieved on 2012-01-18]
- RIZZO A G ET AL: "Treatment of skin neoplasms with polidocanol infiltrations. Our experience (TRATTAMENTO DELLE NEOPLASIE CUTANEE CON INFILTRAZIONI DI POLIDOCANOLO. NOSTRA ESPERIENZA)", TUMORI, IT, vol. 4, no. 3, 1 May 2005 (2005-05-01), pages S197-S198, XP008147715, ISSN: 0300-8916

## Description

The present invention relates to pegylated alcohols for use in the treatment of actinic keratosis.

In general, actinic keratosis (also called "solar keratosis" and "senile keratosis", AK) is a premalignant condition of thick, scaly, or crusty patches of skin consisting of dysplastic keratinocytic lesions. AK is one of the most common conditions treated by dermatologists. It is more common in fair-skinned people. And most important it is associated with those who are frequently exposed to the sun, as it is usually accompanied by solar damage. It is generally accepted that these lesions can progress to squamous cell carcinoma (SSC). Concerning the rate of this transformation there is a controversy in the literature. Annual rates of transformation are ranging from 0.1% - 20%. Nevertheless there is no doubt that these pre-cancerous lesions should be treated in order to prevent manifestation of malignant tumors. In addition lesions are in general treated also for cosmetic purposes and to provide relief from symptoms, such as tenderness or itch.

When skin is exposed to the sun constantly, thick, scaly, or crusty bumps may appear. The scaly or crusty part of the bump is dry and rough. The growths start out as flat scaly areas, and later grow into a tough, wart-like area.

In addition to chronic UV-exposure also infections with HPV has been implicated in the aetiology of AK.

An actinic keratosis site commonly ranges between 2 and 6 millimeters in size, and can be dark or light, tan, pink, red, a combination of all these, or have the same pigment as the surrounding skin. It typically appears on any sun-exposed area, such as the face, ears, neck, scalp, chest, backs of hands, forearms, or lips.

An efficient treatment that can be used to treat larger areas of affected skin and eliminate obvious AK lesions as well as clinically non visible pre-lesions would be beneficial for the patient.

Treatment Options for Actinic Keratosis are inter alia described in McIntyre et al. American Family Physician, 2007, 667 and WO 2010/080345. Uses of polidocanol are inter alia described in Rizzo et al., Official Journal of Societa Italiana di Cancerologica, 2005, 197.

Therefore, it is an object of the present invention to provide alternative treatments for actinic keratosis with improved patient convenience.

This object is solved by Claim 1 of the present invention. Accordingly, molecules of the structure R₁-[O-CH₂-CH₂]ₙ-OH for their use in the treatment of actinic keratosis are provided,
whereby
R₁ is linear C₈-C₁₅ alkyl,
n is an integer from 7 to 11.

Surprisingly it has been found that compounds of this structure can be used for the treatment of actinic keratosis. Without being bound to any theory the inventors believe that this at least partly due to a multiple synergistic effect since many compounds of these structure have found to have at least two of the following effects:
- keratinolytic activity
- analgetic activity

The profound but nevertheless gently keratinolytic activity is responsible for the primary curing effect. The analgetic activity significantly reduces the patients discomfort typically associated during repetitive treatment.

Synthesis methods and instructions of the molecules of the present invention are well-known in the art. The molecules can be synthesized from R₁-OH and either ethylene or propylene oxide.

According to an embodiment of the invention it is preferred that n is 8 to 10.

Preferably the molecules according to the present invention comprise Polidocanol.

The term "Polidocanol" in the sense of the present invention especially relates to and/or includes oligopolyethoxylated dodecanol and/or comprises and/or includes the following chemical structure: 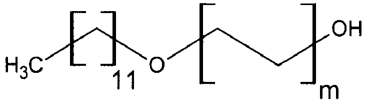 wherein the average m is 9 and the average molecular weight is around 600 Da (in case m is exactly 9 the molecular weight will be 582). Polidocanol can e.g. be made by oligoethoxylation of dodecanol with ethoxylating agents such as ethylene oxide.

Application of a formulation comprising the molecules of the present invention can be carried out by topical application or any form of injection, in particular by epicutaneous injection.

The molecules according to the present invention can be applied topically. Examples for a topic application of a formulation comprising a molecule according to the present invention include a cream, a patch, a salve, a gel, a powder, a dressing, ointment, microemulsions, liposomes, nanoparticles, semi-solid-nanoparticles without or with the use of iontophoresis or transdermal systems.

Preferably the concentration of the molecules according to the present invention is from about 0.001% to about 20% (wt/wt), preferred 0.1 % to 10% and more preferred 1%- 10%. Alternatively, the molecules according to the present invention can be applied by epicutaneous injection. Examples for epicutaneous injection include aqueous solutions, suspensions, oily solutions, emulsions, microemulsions, liposomes, microspheres, nanoparticles and implants. The advantage of cutaneous injections (preferentially into actinic lesion) is the rapid onset of action and that the cytolytic effect is restricted to the targeted tissue. In general by local dermal application the systemic availability of compounds over time is reduced, since drug absorption from epidermal tissue is slow and sustained.

Preferably, each topical application or injection unit of the formulation has a distinct dose of the molecules according to the present invention according to the invention. This dose can reach from about 3 µMol to about 50 mMol, preferred from about 100 µMol to about 10 mMol, per volume of one application.

In case that the application occurs via injection, preferably one injection shot is sized from about 0.10 ml of the formulation to about 5.0 ml, more preferable between 0.5 and 2 ml, preferably around 1 ml.

In case that the application occurs via topical application, preferably per cm² treated area about 0.1 - 5 mg of the formulation, more preferable between 0.5 - 2 mg, preferably around 1 mg is applied.

The present invention furthermore discloses a formulation comprising at least one molecule according to the present invention of the structure R₁-[O-CH₂-CH₂]ₙ-OH for the treatment of actinic keratosis.

The present invention furthermore discloses a process, comprising: administering molecules according to the present invention of the structure R₁-[O-CH₂-CH₂]ₙ-OH to a human in an amount effective for treating actinic keratosis

The claimed molecules can be combined with an additional keratolytic agent in order to support the therapeutic effect. The keratolytic agent could simply be a chemical peel like compound like salicylic-acid ore could have a more efficient keratino-cytolic activity like retinoids.

Additional details, characteristics and advantages of the object of the invention are disclosed in the subclaims and the following description of the respective figure and example.

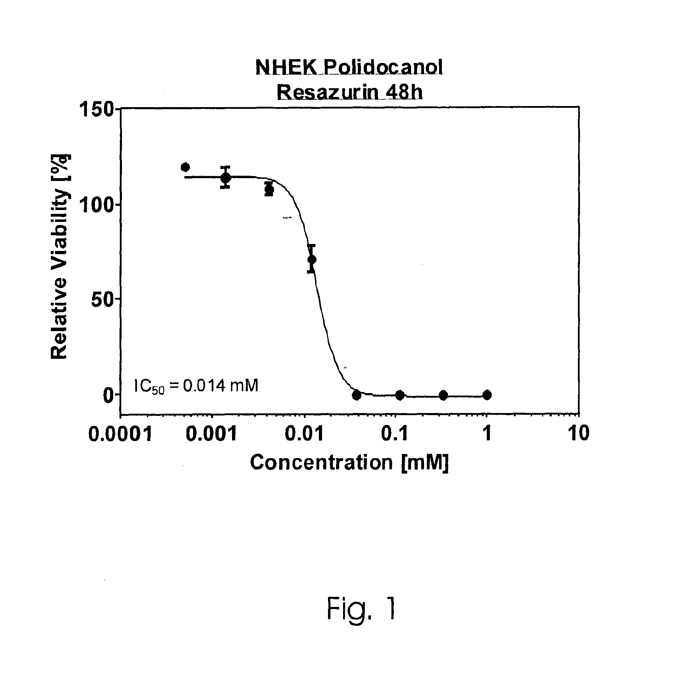

Fig. 1 is a diagram showing the relative viability vs. the concentration for the compound according to Example 1 of the present invention on NHEK-cells

### EXAMPLE I:

Example 1 refers to Polidocanol, whose structure is given above.

By using primary, normal human epidermal keratinocytes (NHEK, Fig. 1) the cytolytic potential of the drug compound was investigated.

The viability of the cells was monitored by their metabolic activity using the Resazurin - assay. Eight different concentrations (up to 1 mM) of polidocanol were investigated. Viability of the cells was plotted against the different concentration of test compound.

From the resulting sigmoid-curve the IC50 value for the cytolytic effect was determined and given in the respective figure. Fig. 1 depicts the relative viability of NHEK-cells vs. the concentration of test-compound using metabolic activity as read-out. The obtained data show a clear negative-impact of suggested drug compound on the viability of the cells.

The particular combinations of elements and features in the above detailed embodiments are exemplary only. The scope of the invention is defined in the claims.

## Claims

1. Molecules of the following structure
R₁-[O-CH₂-CH₂]ₙ-OH
for use in the treatment of actinic keratosis, whereby
R₁ is linear C₆ - C₁₅ - alkyl
n is an integer from 7 to 11

2. Molecules for use according to claim 1, whereby n is 8 to 10.

3. Molecules for use according to Claim 1 or 2, whereby R₁ is linear C₁₀ to C₁₂ -alkyl

4. Molecules for use according to any of the claims 1 to 3, whereby the molecules comprise polidocanol.

5. Molecules for use according to any of the claims 1 to 4, whereby the molecules are applied topically or in form of an injection

6. Molecules for use according to any of the claims 1 to 5, whereby the dose of molecules according to the present invention per one application is between from about 3 µMol to about 50 mMol.

## Patentansprüche

1. Moleküle mit der folgenden Struktur
R₁-[O-CH₂-CH₂]ₙ-OH
zur Behandlung von aktinischer Keratose, wobei
R₁ ein lineares C₈ bis C₁₅-Alkyl ist, und
n eine ganze Zahl von 7 bis 11 ist.

2. Moleküle nach Anspruch 1, wobei n 8 bis 10 beträgt.

3. Moleküle nach Anspruch 1 oder 2, wobei R₁ ein lineares C₁₀- bis C₁₂-Alkyl ist.

4. Moleküle nach einem der Ansprüche 1 bis 3, wobei die Moleküle Polidocanol aufweisen.

5. Moleküle nach einem der Ansprüche 1 bis 4, wobei die Moleküle topisch oder in Form einer Injektion angewendet werden.

6. Moleküle nach einem der Ansprüche 1 bis 5, wobei die Dosis der erfindungsgemäßen Moleküle pro Anwendung im Bereich von etwa 3 µMol bis etwa 50 mMol liegt.

## Revendications

1. Molécules ayant la structure suivante
R₁[O-CH₂-CH₂]ₐ-OH
pour utilisation dans le traitement de la kératose actinique où
R₁ est un alkyle linéaire C₉-C₁₅ n est un nombre entier de 7 à 11.

2. Molécules pour utilisation selon la revendication 1 où n est de 8 à 10.

3. Molécules pour utilisation selon la revendication 1 ou 2, où R₁ est un alkyle linéaire C₁₀-C₁₂.

4. Molécules pour utilisation selon une quelconque des revendications 1 à 3, où les molécules comprennent le polydocanol.

5. Molécules pour utilisation selon une quelconque des revendications 1 à 4, où les molécules sont appliquées de manière topique ou sous forme d'injection.

6. Molécules pour utilisation selon une quelconque des revendications 1 à 5 où la dose des molécules selon la présente invention par application est entre environ 3 µMol à environ 50 mMol.
